# EUROPÄISCHE PATENTSCHRIFT

(11) **EP 3 487 430 B1**
(45) Veröffentlichungstag und Bekanntmachung des Hinweises auf die Patenterteilung: **02.09.2020**
(21) Anmeldenummer: 17742240.9
(22) Anmeldetag: 20.07.2017
(51) Int. Cl.: A61B 17/70, F16B 35/04

(54) **PEDIKELSCHRAUBENSYSTEM MIT EINER VERRIEGELUNGSSCHRAUBE MIT GEWINDEANSCHNITT**
PEDICLE SCREW SYSTEM COMPRISING A LOCKING SCREW WITH THREADED CHAMFER
SYSTÈME DE VIS PÉDICULAIRE MUNI D'UNE VIS DE VERROUILLAGE COMPRENANT UNE PARTIE FILETÉE

(30) Priorität: 21.07.2016 DE 102016113495
(43) Veröffentlichungstag der Anmeldung: 29.05.2019
(73) Patentinhaber: Aesculap AG, 78532 Tuttlingen (DE)
(72) Erfinder: LINDNER, Stephan, 78573 Wurmlingen (DE); KRÜGER, Sven, 78647 Trossingen (DE); HAAS, Alexander, 78166 Donaueschingen (DE)
(74) Vertreter: Winter, Brandl, Fürniss, Hübner, Röss, Kaiser, Polte - Partnerschaft mbB
(86) Internationale Anmeldenummer: PCT/EP2017/068332
(87) Internationale Veröffentlichungsnummer: WO 2018/015482

(56) Entgegenhaltungen:
- EP-A1- 2 334 938
- EP-A1- 2 674 123
- DE-A1-102014 108 225

## Beschreibung

Die vorliegende Erfindung betrifft ein Knochenschraubensystem, insbesondere ein Pedikelschraubensystem, das eine Knochenschraube, insbesondere eine Pedikelschraube, mit einer insbesondere am Schraubenkopf vorgesehenen Aufnahmehülse oder Tulpe, die eine Aufnahme für einen Längsträger zur chirurgischen Verbindung benachbarter Knochenschrauben bzw. Pedikelschrauben ausbildet und mit einem Tulpengewinde versehen ist, und eine in das Tulpengewinde der Aufnahmehülse einschraubbare Klemmschraube mit einem Klemmschraubengewinde zur klemmenden Verriegelung des Längsträgers in der Aufnahme aufweist, wobei das Klemmschraubengewinde mit einem Gewindeanschnitt versehen ist. Ferner betrifft die Erfindung eine entsprechende Klemmschraube für das Knochenschraubensystem.

Knochen- sowie Pedikelschrauben sind aus dem Stand der Technik bekannt. Sie dienen beispielsweise einer dorsalen Stabilisierung der Wirbelsäule mittels transpedikulärer Verschraubung. Dabei werden Pedikelschrauben in den Pedikeln jeweils benachbarter Wirbel platziert, worauf eine winkelstabile Verbindung zwischen den jeweils axial übereinander angeordneten Pedikelschrauben und einem sich axial erstreckenden Längsträger oder Steg geschaffen wird, der mittels einer Klemmschraube hinsichtlich der Knochen- oder Pedikelschraube verriegelt wird. Die Pedikelschrauben und Längsträger bilden dabei ein Wirbel-Stabilisierungssystem.

Eine Pedikelschraube weist üblicherweise einen in axialer Richtung verlaufenden Schraubenschaft mit Außengewinde auf, an den sich schraubenkopfseitig eine Aufnahmehülse, die sogenannte Tulpe, anschließt. Diese ist im Wesentlichen U-förmig ausgebildet mit gegenüberliegenden Wandabschnitten (Hülsenflanken) und einem dazwischen ausgebildeten, in radialer Richtung verlaufenden Spalt als Aufnahme für den Längsträger oder Steg. Die Tulpe ist mit einem in axialer Richtung verlaufenden Tulpengewinde versehen. Der Längsträger wird in radialer Richtung in den Spalt der Tulpe eingelegt und mittels der Klemmschraube, die auch als Verriegelungsschraube oder Setscrew bezeichnet wird und mit dem Tulpengewinde verschraubt wird, fixiert.

Bei Pedikelschraubensystemen ist das Problem bekannt, dass es nicht immer einfach ist, die Klemmschraube in den Tulpenkopf einzusetzen. Grund dafür ist unter anderem ein geringes Gewindespiel zwischen dem Tulpengewinde der Pedikelschraube und dem Klemmschraubengewinde der Klemmschraube. Durch Vorsehen eines größeren Gewindespiels ist das Problem nicht in zufriedenstellender Weise zu lösen, da das Gewindespiel aufgrund der geringen Umgreifung der beiden Gewindeteile sowie einer sicheren Klemmwirkung gering sein muss und andernfalls die Funktionssicherheit und die Klemmung beeinträchtigt wären.

Das Problem wird in der Regel außerdem dadurch verstärkt, dass bei einer Manipulation durch Instrumente Kräfte auf das Implantat ausgeübt werden. Diese können dazu führen, dass sich der Tulpenkopf elastisch verformt, insbesondere nach innen deformiert. Zum Beispiel kann es beim Ansetzen der Klemmschraube an einer Pedikelschraube zum Fixieren des Längsträgers durch Einwirken von Kräften auf die Pedikelschraube und insbesondere auf deren Tulpe, beispielsweise bei einer Manipulation von Wirbelkörpern oder bei einem Andrücken des Längsträgers, zur Verformung der Tulpe kommen. Dadurch verändert sich die Geometrie des Tulpengewindes, zum Beispiel verkleinert sich dessen Innendurchmesser, was ein Einschrauben der Klemmschraube zusätzlich erschwert oder verhindert.

Grundsätzlich können zwei Arten von Verformungen auftreten, solche, bei denen das Tulpengewinde in Richtung des Klemmschraubengewindes verformt wird, und solche von entgegengesetzter Richtung. Besonders problematisch bei erstgenannten Verformungen sind solche Verformungen, die ein Ansetzten der Setscrew am Tulpengewinde verhindern, weil die Gewinde nicht mehr ineinander greifen können. Jedoch selbst bei einer geringgradigen Verformung, die noch ein Einschrauben zulässt, kann es infolge der Verformung der Tulpe zu einem Verkanten der Setscrew relativ zur Achse der Tulpe kommen. Das kann dazu führen, dass der Gewindeanschnitt, insbesondere der der Setscrew, beschädigt wird. Es kann zum sogenannten "crossthreading" kommen, was bedeutet, dass die Setscrew soweit zur Längsachse des Schraubenschafts und dessen Außengewindes verkantet ist, dass der Gewindeanfang der Setscrew, also der Einlaufgewindegang oder die Einlaufgewindegänge, in den falschen Gewindegang des Innengewindes der Tulpe eingreift bzw. eingreifen, was zu einer Beschädigung der Gewinde bis hin zur Unbrauchbarkeit der Setscrew und/oder der Pedikelschraube führen kann.

Aus der US 2014/0350605 A1 ist bekannt ein Pedikelschraubensystem, bei dem eine Klemmschraube einen angefasten Kopfbereich aufweist, der ein Ansetzen in einem in der Tulpe ausgebildeten Innengewinde erleichtert. Zusätzlich weist das Innengewinde der Tulpe einen verkürzt ausgebildeten ersten Gewindegang auf, so dass die Klemmschraube mittels des angefasten Kopfbereichs zunächst ohne Gewindeeingriff in die Tulpe eingeführt und zentriert werden kann und nachfolgend in dieser Lage gedreht wird, wobei Gewindeeingriff entsteht. Die Verkürzung des Gewindegangs kann unter Umständen in nachteiliger Weise eine Schwächung des Gewindes darstellen.

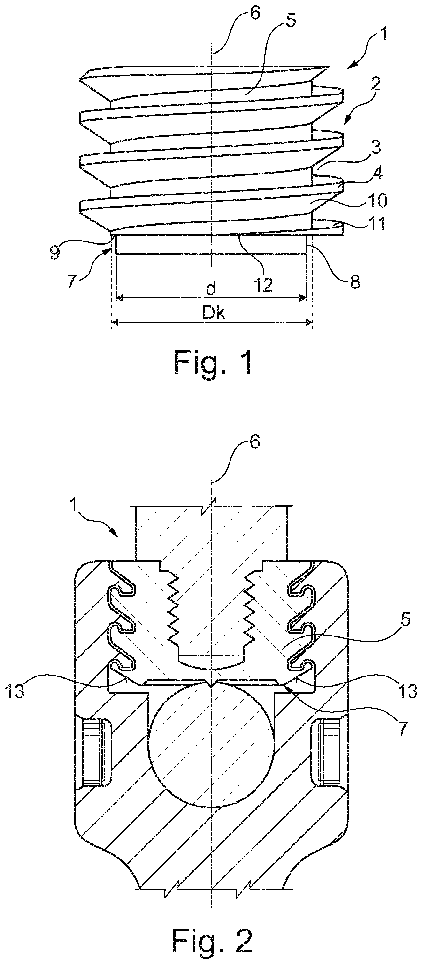

Aus der US 7,780,706 B2 ist ein ähnliches Pedikelschraubensystem, bei dem eine Klemmschraube im Bereich ihres Gewindeeinlaufs mit einem Absatz versehen ist. Der Absatz besitzt einen Durchmesser, der geringer ist als der Gewindeinnendurchmesser des Klemmschraubengewindes. Zum Klemmschraubengewinde hin endet der Absatz mit einer Schulter, die sich quer zur Längsachse/Schraubenachse der Klemmschraube erstreckt. Infolge dieser Ausrichtung der Schulter ist das Gewinde orthogonal zur Längsachse der Klemmschraube angeschnitten. Dadurch ist der erste Gewindegang einlaufseitig angeschnitten, derart, dass das sich radial nach außen erstreckende Gewindeprofil (die durch Gewindeflanken begrenzte Gewindeerhebung) in umfänglicher Richtung beginnend mit einer Spitze kontinuierlich keilförmig an Stärke zunimmt, bis die Schulter nicht mehr eine der Gewindeflanken des ersten Gewindegangs schneidet (Figur 1 zeigt eine derartige bekannte Klemmschraube in einer seitlichen Ansicht). Man kann auch sagen, dass sich der Gewindeanschnitt bei dieser Klemmschraube in radialer Richtung mit konstanter Tiefe in die Klemmschraube eingebracht ist und in umfänglicher Richtung betrachtet in axialer Richtung in den Gewindegang vordringt. Bei einem solchen Anschnitt des Gewindes ist besonders nachteilig, dass die angeschnittene Gewindeerhebung abschnittsweise, insbesondere nahe der Spitze, sehr dünn und damit empfindlich ist. Sie kann sich beispielsweise beim Einschrauben leicht verformen.

Aus der US 8,257,402 B2 ist ein Verschluss für ein einen Stab aufnehmendes chirurgisches Implantat in Form einer Klemmschraube mit Außengewinde bekannt, bei der eingangsseitig des Außengewindes eine Phase ausgebildet ist. Durch die Phase ist die Klemmschraube stirnseitig mit einer umlaufenden Abschrägung versehen, die ein Einsetzen in ein Innengewinde des Implantats erleichtert. Auch hier ist der erste Gewindegang einlaufseitig angeschnitten, derart, dass das sich radial nach außen erstreckende Gewindeprofil (die durch Gewindeflanken begrenzte Gewindeerhebung) in umfänglicher Richtung beginnend mit einer Spitze kontinuierlich keilförmig an Stärke zunimmt, bis die Phase nicht mehr eine der Gewindeflanken des ersten Gewindegangs schneidet (Figur 2 zeigt eine derartige bekannte Klemmschraube in einer seitlichen Ansicht). Auch hier kann man sagen, dass der Gewindeanschnitt mit konstanter radialer Tiefe eingebracht oder ausgebildet ist. Es ist ebenfalls nachteilig, dass die angeschnittene Gewindeerhebung abschnittsweise, insbesondere nahe der Spitze, sehr dünn und damit empfindlich ist. Sie kann sich beispielsweise beim Einschrauben leicht verformen.

Eine Klemmschraube mit den Merkmalen des Oberbegriffs des Anspruchs 1 ist aus der EP 2 674 123 A1 bekannt.

Da Verformungen der Pedikelschraube und insbesondere der Tulpe nicht immer sicher vermieden werden können, liegt ausgehend vom vorstehend beschriebenen Stand der Technik der Erfindung die Aufgabe zugrunde, ein Knochenschraubensystem, insbesondere ein Pedikelschraubensystem, bereit zu stellen, das hinsichtlich solcher Verformungen robust ist und ein Ansetzen einer Klemmschraube an einer Knochen- oder Pedikelschraube erleichtert und sicherer macht, ohne dass dazu zusätzliche Elemente oder Instrumente wie Führungshülsen etc. notwendig sind oder die Sicht des Operateurs eingeschränkt wird. Die Klemmschraube soll sich auch dann noch einfach einschrauben lassen, wenn der Tulpenkopf durch von außen wirkende Kräfte elastisch nach innen verformt wird. Des Weiteren soll das System robust und stabil sein, insbesondere soll das Risiko einer Beschädigung des Klemmschraubengewindes, insbesondere dessen Gewindeeinlaufs, beim Einschrauben reduziert sein.

Diese Aufgabe wird nach der vorliegenden Erfindung durch eine Klemmschraube gemäß Anspruch 1 bzw. durch ein Knochenschrauben- bzw. Pedikelschraubensystem gemäß dem nebengeordneten Anspruch gelöst. Erfindungsgemäß weist hierbei das Klemmschraubengewinde einlaufseitig einen Gewindeanschnitt auf, an dem das Klemmschraubengewinde in radialer Richtung reduziert oder zurückgenommen ist.

Die vorliegende Beschreibung erfolgt mit Bezug auf eine Knochenschraube. Die Erfindung betrifft jedoch insbesondere eine Pedikelschraube. Die Bezeichnung "Knochenschraube" ist daher als auf eine Pedikelschraube gerichtet zu verstehen und umgekehrt.

Anders als beim vorstehend beschriebenen Stand der Technik ist der Gewindeanschnitt nicht derart ausgebildet, dass der erste Gewindegang in axialer Richtung angeschnitten ist, sondern derart, dass der erste Gewindegang in radialer Richtung angeschnitten ist. Man kann auch sagen, dass das Gewinde bzw. die Gewindeerhebung durch den (radialen) Gewindeanschnitt abgeflacht oder zurückgenommen ist, insbesondere in radiale Richtung zur Mittellängsachse der Klemmschraube hin abgeflacht oder zurückgenommen ist. Umgekehrt betrachtet ist der Gewindeanschnitt nach der Erfindung in axialer Richtung auslaufend ausgebildet, während er beim vorstehend beschriebenen Stand der Technik in seinem umfänglichen Verlauf in axialer Richtung konstant ist. Die kleinste Erstreckung des Gewindeanschnitts in axialer Richtung ist nach der Erfindung vorzugsweise nicht kleiner als 0,5 mm.

Durch die Erfindung kann der Vorteil erzielt werden, dass die Gewindespitze bzw. der Abstand zwischen den Gewindeflanken im Bereich des Gewindeanschnitts nicht verjüngt und damit geschwächt wird. Vielmehr bleibt der Gewindequerschnitt in axialer Richtung auch im Bereich des Gewindeanschnitts vollständig erhalten. So ist der bei einem Einschrauben der Klemmschraube in die Tulpe der Knochenschraube zuerst mit dem Tulpengewinde in Eingriff gelangende Teil des Klemmschraubengewindes besonders stabil und robust, so dass Verformungen davon verhindert oder zumindest im Wesentlichen verringert werden können. Gleichwohl ist bei dem erfindungsgemäßen Knochenschraubensystem ein Einschrauben der Klemmschraube in das Tulpengewinde infolge des Gewindeanschnitts besonders einfach möglich.

Nach der Erfindung ist derart auf der Einlaufseite des Tulpengewindes eine Führung für eine in das Gewinde einzuschraubende Klemmschraube vorgesehen, ohne dass das Gewinde durch die Führung geschwächt würde. Diese Führung ist so ausgebildet, dass die Klemmschraube auch bei verformter Tulpe in einfacher Weise an deren Gewindeeinlauf positioniert und eingeschraubt werden kann. Auf diese Weise ist trotz Verformung ein gegenseitiges Eingreifen von Tulpengewinde und Klemmschraubengewinde sichergestellt. Anders ausgedrückt wird durch den Gewindeanschnitt zwischen der Klemmschraube und der Tulpe ein Abschnitt mit einem bestimmten Radialspiel und ohne gegenseitigen Gewindeeingriff bereitgestellt, über das Verformungen der Tulpe ausgeglichen werden können. Die Klemmschraube kann so auch bei verformter Tulpe einfach und sicher an dieser angeordnet und relativ zum Tulpengewinde positioniert werden. Es ist ein besonderer Vorteil, dass die Klemmschraube durch einen Operateur auch ohne Sicht korrekt an der Pedikelschraube zu positionieren ist und vor einem gegenseitigen Gewindeeingriff geführt und gestützt ist. Es bedarf daher keiner besonderen Mühe oder zusätzlicher Instrumente, um die Klemmschraube bei einem Ansetzen am Tulpengewinde mit der geforderten Genauigkeit in die zum Einschrauben in die Tulpe bestimmte Position zu bringen. Der durch den Gewindeanschnitt begrenzte oder definierte Sitz der Klemmschraube ist für den Operateur auch haptisch zu erfassen, so dass ein bestimmungsgemäßes Verschrauben vereinfacht und Fehlpassungen der Klemmschraube zur Pedikelschraube sicher vermieden werden. Es ist nicht länger erforderlich, dass ein Operateur besonderes Augenmerk auf das Ansetzen der Setscrew richtet, was erhebliche Erleichterung und Zeitvorteile mit sich bringt. Wird die mit dem Gewindeanschnitt geführte Klemmschraube nun um ihre eigene Gewindeachse verdreht, gelangen die Gewinde von Klemmschraube und Tulpe in Eingriff. Die Klemmschraube wird infolge ihrer Schraubbewegung auch in axialer Richtung vorbewegt. Aufgrund der axialen Verlagerung der Klemmschraube in Richtung der Pedikelschraube kann eine Verformung der letzteren, insbesondere der Hülsenflanken, zurückgestellt werden. Im Ergebnis kann mit der Erfindung eine Klemmschraube einfach an einer unter Last stehenden und daher verformten Pedikelschraube angesetzt und mit dieser verschraubt werden.

Der Gewindeanschnitt weist dabei eine umfängliche Mantelfläche auf. Diese Mantelfläche kann insbesondere parallel zur Mittellängsachse der Klemmschraube ausgebildet sein. Die Mantelfläche kann nach der Erfindung als eine Art Führungsfläche wirken oder ausgebildet sein, mittels der die Klemmschraube vor einem eigentlichen gegenseitigen Gewindeeingriff mit dem Tulpengewinde relativ zur Tulpe positioniert und geführt sein kann und insbesondere in die Aufnahme auch bei verformter Tulpe eingeführt werden kann.

Ferner weist der Gewindeanschnitt einen Übergangsabschnitt (zwischen dem angeschnittenen Gewinde und dem nicht angeschnittenen Gewinde) auf, der je nach Betrachtungsrichtung als Einlaufabschnitt oder Auslaufabschnitt wirkt. In diesem Übergangsabschnitt weist die umfängliche Mantelfläche einen variierenden radialen Abstand von der Mittellängsachse der Klemmschraube auf. Er bildet demnach eine Art Übergang vom Gewindeanschnitt zum Klemmschraubengewinde aus und ist insbesondere auf der zum Klemmschraubengewinde hin gerichteten Seite des Gewindeanschnitts angeordnet.

Der radiale Abstand der Mantelfläche von der Mittellängsachse entspricht auslaufseitig des Übergangsabschnitts dem Gewindeaußenradius. Auf diese Weise ist ein weicher Übergang vom Gewindeanschnitt zum Klemmschraubengewinde ausgebildet, was ein einfaches Einschrauben der Klemmschraube ohne Verhaken oder Verklemmen ermöglicht. Der radiale Abstand der Mantelfläche von der Mittellängsachse einlaufseitig des Übergangsabschnitts (d.h. in einem bestimmten Winkelbereich) ist ferner kleiner als der oder vorzugsweise gleich dem Gewindekernradius. In anderen Worten ausgedrückt ist der Gewindekern in Umfangsrichtung vor dem Anschnitt des Außengewindes (Übergangsabschnitt), vorzugsweise über einen Bereich von maximal 90 - 180° oder weniger, abgeflacht. Auf diese Weise ist die Klemmschraube mit einem durch den Gewindeanschnitt gebildeten Führungsabschnitt versehen, dessen Durchmesser kleiner ist als der Kerndurchmesser des Tulpengewindes, so dass auch bei verformter Tulpe ein einfaches Ansetzen der Klemmschraube und Ineingriffbringen der beiden Gewinde möglich ist.

Vorteilhafte Ausführungsformen der Erfindung sind in den Unteransprüchen beansprucht und werden nachfolgend näher erläutert.

Eine Ausführungsform der Erfindung ist dadurch gekennzeichnet, dass sich der radiale Abstand der Mantelfläche des Übergangsabschnitts von der Mittellängsachse im umfänglichen Verlauf kontinuierlich und/oder konstant ändert. Dies begünstigt eine allmähliche Rückverformung der Tulpe im Falle deren Verformung. Die Änderung kann insbesondere linear oder bogenförmig oder kreisbogenförmig erfolgen.

In einer bevorzugten Ausführungsform kann die Klemmschraube, infolge der Änderung des radialen Abstands der Mantelfläche von der Längsachse, stirnseitig ein nicht kreisrundes (Querschnitts-)Profil ausbilden. Die Klemmschraube weist dabei aufgrund des nicht kreisrunden Profils eine Einsetzstellung mit einer Einsetzfunktion und eine Spreizstellung mit einer Spreizfunktion auf. Im speziellen entspricht der radiale Abstand der Mantelfläche auslaufseitig des Übergangsabschnitts dem Gewindeaußenradius (Nennradius des Gewindes) und ist größer als der radiale Abstand der Mantelfläche von der Mittellängsachse einlaufseitig des Übergangsabschnitts. Hierdurch ergeben sich stirnseitig der Klemmschraube unterschiedliche Abstände von sich in radialer Richtung diametral gegenüberliegenden Punkten auf der Mantelfläche bzw. von einem Punkt auf der Mantelfläche zu einem Punkt auf der Fläche des Gewindeaußenradius der ersten Gewindeerhebung. Die Verbindungslinien der Punkte schneiden dabei jeweils die Mittellängsachse. Der Abstand von zwei sich diametral gegenüberliegenden Punkten wird im Folgenden als Durchmesser bezeichnet. Die Klemmschraube weist also stirnseitig zumindest zwei unterschiedliche Durchmesser auf. Vorzugsweise liegt ein maximaler Durchmesser dabei orthogonal zu einem minimalen Durchmesser. Die Klemmschraube weist im Zusammenwirken mit der Tulpe der Pedikelschraube zwei unterschiedliche Stellungen mit zwei unterschiedlichen Funktionen auf. In der ersten Stellung bzw. Einsetzstellung mit Einsetzfunktion liegen an den Flanken der (verformten) Tulpe bzw. den Tulpenarmen die Mantelflächen außerhalb des Übergangsbereichs an. Die Klemmschraube ist über den einhergehend kleineren Durchmesser (kleiner als der maximale Durchmesser) in die Tulpe einsetzbar. Anschließend wird die Klemmschraube mittels einer Drehbewegung in die zweite Stellung bzw. Spreizstellung mit Spreizfunktion in die Tulpe eingedreht. Durch die Drehbewegung wird der Übergangsbereich mit dem größer werdenden radialen Abstand der Mantelfläche zwischen die beiden Flanken der Tulpe eingedreht. Die Vergrößerung des zwischen den Flanken der Tulpe liegenden Durchmessers spreizt dabei die Flanken der Tulpe. In der zweiten Stellung liegt also der Übergangsbereich, insbesondere der auslaufseitige Übergangsbereich, oder die radiale Außenfläche der ersten Gewindeerhebung an der Flanke der Tulpe an. Die Spreizstellung ist vorzugsweise um 90 Grad zu der Einsetzstellung drehversetzt. Hierdurch wird ein Einschrauben einer Klemmschraube bei geringen Toleranzen bzw. bei einem sehr geringen Gewindespiel erleichtert, ohne einen maximalen Durchmesser ("Aufhalte-Durchmesser") zu mindern.

Der Übergangsabschnitt kann sich insbesondere in Umfangsrichtung über einen Winkelabschnitt α zwischen ca. 20° und ca. 180°, vorzugsweise zwischen ca. 40° und ca. 135°, bevorzugter zwischen ca. 60° und ca. 90° erstrecken. So wird ein genügend großer Führungsbereich gebildet, der ein Ansetzen der Klemmschraube auch bei verhältnismäßig großen Verformungen der Klemmschraube ermöglicht.

Nach einer Ausführungsform der Erfindung ist der Gewindeanschnitt in einem Teilbereich teilzylinderförmig mit konstantem radialem Abstand von der Mittellängsachse ausgebildet. In diesem Teilbereich kann der radiale Abstand von der Mittellängsachse insbesondere kleiner als der Gewindekernradius sein. Der Teilbereich mit konstantem radialem Abstand kann sich in umfänglicher Richtung insbesondere über einen Winkelbereich β von mindestens 90°, vorzugsweise von 90° bis 180° erstrecken.

Eine Ausführungsform der Erfindung ist dadurch gekennzeichnet, dass der Gewindeanschnitt dort endet, wo die erste vollständige Gewindeerhebung des Gewindegangs beginnt bzw. wo die Gewindespitze ihre volle / normale Breite aufweist. Auf diese Weise ist der erste, einen Gewindeeingriff mit dem Tulpengewinde ausbildende Bereich des Klemmschraubengewindes sehr robust und nicht versehentlich verformbar ausgebildet.

Es ist von besonderem Vorteil, wenn der Gewindeanschnitt der Steigung des Klemmschraubengewindes folgend ausgebildet ist. Man kann dann auch sagen, dass der Gewindeanschnitt mit gleicher Steigung wie das Klemmschraubengewinde ausgebildet ist. Auf diese Weise wird das Klemmschraubengewinde an keiner Stelle in axialer Richtung angeschnitten und geschwächt, so dass es besonders robust ist.

Zusammenfassend kann man sagen, dass durch die Erfindung ein Gewindeanschnitt bereitgestellt wird, der dieser seinen Startpunkt an der Stelle des Gewindeauslaufes haben kann, also an dem Punkt, ab dem sich die Flankenbreite des Gewindes reduziert. Der Gewindeanschnitt kann an dem Punkt beginnen, ab dem die Flankenbreite durch den Gewindeauslauf kleiner werden würde, wenn es keinen Anschnitt gäbe. Der Gewindeanschnitt kann dann mittels des Übergangsbereiches auf einen Kerndurchmesser kleiner dem Gewindekerndurchmesser reduziert sein. Dieser (reduzierte) Kerndurchmesser kann sich über einen Abschnitt von min. 90° erstrecken. Der besagte Kerndurchmesser muss nicht rund sein, vielmehr sollen alle Abschnitte bezogen auf den Durchmesser unterhalb des Gewindekerndurchmessers liegen.

Die Erfindung schafft insbesondere die folgenden Vorteile:
- Robuster Gewindeanschnitt
- Einfaches Einschrauben des Verriegelungsschraube
- Keine Schwächung des Gewindes

Weitere Merkmale und Vorteile der vorliegenden Erfindung ergeben sich aus der folgenden beispielhaften und nicht beschränkenden Beschreibung der Erfindung anhand eines Pedikelschraubensystems als Beispiel für ein Knochenschraubensystem anhand von Figuren. Diese sind lediglich schematischer Natur und dienen nur dem Verständnis der Erfindung. Dabei zeigen:
Fig. 1 eine erste Klemmschraube nach dem Stand der Technik in einer Frontansicht;
Fig. 2 eine zweite Klemmschraube nach dem Stand der Technik in einer Frontansicht;
Fig. 3 eine Pedikelschraube eines erfindungsgemäßen Systems in einer Seitenansicht und gekennzeichneten Verformungen;
Fig. 4 die Pedikelschraube der Fig. 3 mit angesetzter Klemmschraube;
Fig. 5 eine Klemmschraube nach der Erfindung in einer perspektivischen Ansicht;
Fig. 6 die Klemmschraube der Fig. 5 in einer Ansicht von unten;
Fig. 7 eine Variante der Klemmschraube der Fig. 5; und
Fig. 8 eine weitere Variante der Klemmschraube der Fig. 5.

Figur 1 zeigt eine Klemmschraube (set screw) 1 nach dem Stand der Technik in einer seitlichen Ansicht. Die Klemmschraube 1 ist als Madenschraube ausgebildet und weist ein als Außengewinde ausgebildetes Klemmschraubengewinde 2 auf. Dieses ist als eingängiges Gewinde ausgebildet und besitzt einen Gewindeeinschnitt 3 und eine Gewindeerhebung 4. Der Gewindeeinschnitt 3 und die Gewindeerhebung 4 laufen spiralförmig um einen Klemmschraubengrundkörper 5 um. Gezeigt in Figur 1 ist auch eine Mittellängsachse 6 der Klemmschraube 1. Einlaufseitig des Klemmschraubengewindes 2 ist die Klemmschraube mit einem Gewindeanschnitt 7 versehen. Dieser ist in Form eines Absatzes 8 ausgebildet und besitzt einen Durchmesser d, der geringer ist als der Gewindekerndurchmesser Dₖ des Klemmschraubengewindes 2. Zum Klemmschraubengewinde 2 hin endet der Absatz 8 mit einer Schulter 9, die sich quer zur Längsachse/Schraubenachse 6 der Klemmschraube 1 erstreckt. Infolge dieser Ausrichtung der Schulter 9 ist das Gewinde 2 orthogonal zur Längsachse 6 der Klemmschraube 1 angeschnitten. Dadurch ist der erste Gewindegang einlaufseitig angeschnitten, derart, dass das sich radial nach außen erstreckende Gewindeprofil (die durch Gewindeflanken 10, 11 begrenzte Gewindeerhebung 4) in umfänglicher Richtung beginnend mit einer Spitze 12 kontinuierlich keilförmig an Stärke zunimmt, bis die Schulter 9 nicht mehr eine der Gewindeflanken 10, 11 des ersten Gewindegangs schneidet.

Figur 2 zeigt eine andere aber ähnliche bekannte Klemmschraube 1, bei der ein Gewindeanschnitt 7 nicht mit einer sich orthogonal zur Mittellängsachse 6 erstreckenden Schulter 9, sondern mit einer umlaufenden Abschrägung 13 versehen ist. Auch hier ist der erste Gewindegang einlaufseitig angeschnitten, derart, dass das sich radial nach außen erstreckende Gewindeprofil (die durch Gewindeflanken 10, 11 begrenzte Gewindeerhebung 4) in umfänglicher Richtung beginnend mit einer Spitze kontinuierlich keilförmig an Stärke zunimmt, bis die umlaufende Abschrägung 13 nicht mehr eine der Gewindeflanken 10, 11 des ersten Gewindegangs schneidet.

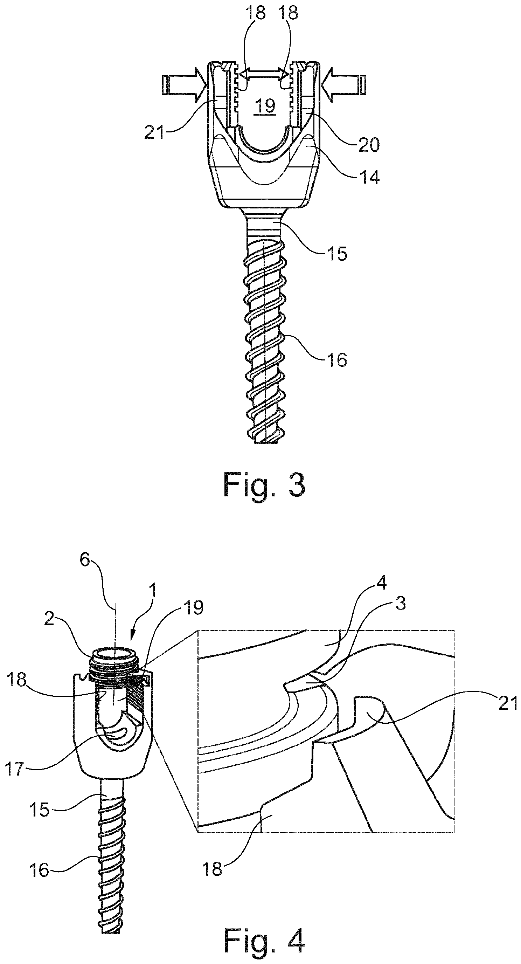

Die Figuren 3 und 4 verdeutlichen das Problem einer Verformung einer Tulpe 14 oder Aufnahmehülse 14 einer Pedikelschraube 15. Diese ist auf der der Aufnahmehülse 14 gegenüberliegenden Seite mit einem Außengewinde 16, auch als Knochengewinde 16 bezeichnet, versehen, mit dem sie in einen Pedikelkanal eines Wirbels (als Beispiel für einen Knochen) eingeschraubt werden kann. Auf der Seite der Tulpe 14 ist die Pedikelschraube 15 zu diesem Zweck mit einem Schraubwerkzeugeingriff 17 versehen. Die Tulpe 14 weist eine im Wesentlichen U-förmige Gestalt mit einem in axialer Richtung darin eingebrachten und ein Tulpengewinde 18 aufweisenden Loch oder Schlitz 19 auf. Anders ausgedrückt kann die Tulpe 14 ausgebildet sein, indem von einem Hohlzylinder auf radial einander gegenüberliegenden Seiten in axialer Richtung Material entfernt wird und das Loch des Hohlzylinders mit dem Tulpengewinde 18 versehen wird. Vom Hohlzylinder verbleiben zwei radial einander gegenüberliegenden Hülsenwandabschnitte oder Hülsenflanken 20, 21, deren einander zugewandte Innenflächen das Loch 19 begrenzen und mit dem Tulpengewinde 18 versehen sind. Die Hülsenwandabschnitte 20, 21 dienen unter anderem als Werkzeugkontaktflächen für ein nicht gezeigtes Handhabungswerkzeug und können sich aufgrund ihrer schlanken Gestalt insbesondere unter Krafteinwirkung durch das Werkzeug in radialer Richtung verformen. Derartige Verformungen sind in Figur 3 angedeutet und stehen einem Ansetzen und Einschrauben der Klemmschraube 1, wie sich aus Figur 4 ergibt, hindernd entgegen.

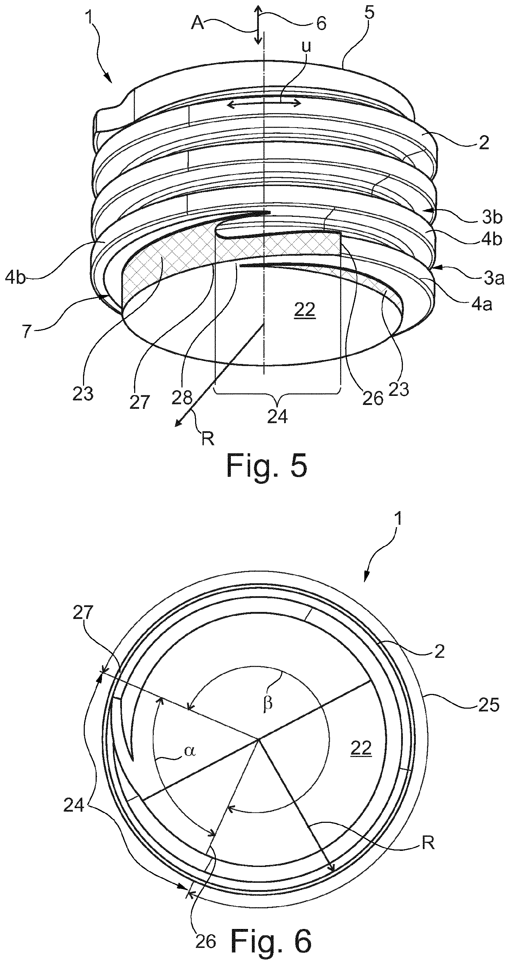

Figur 5 zeigt die Klemmschraube 1 eines erfindungsgemäßen Pedikelschraubensystems oder Knochenschraubensystems in einer perspektivischen Ansicht, bei der die untere Stirnseite 22 zu erkennen ist. Die Klemmschraube 1 ist als Madenschraube ausgebildet und weist auf der der Stirnseite 22 gegenüberliegenden in der Figur 5 nicht erkennbaren Stirnseite einen Werkzeugeingriff für ein Schraubwerkzeug auf. Die Klemmschraube 1 besitzt ein eingängiges Klemmschraubengewinde 2 in Form eines Außengewindes. Dessen Gewindeerhebung 4 (mit erster Gewindeerhebung 4a und zweiter Gewindeerhebung 4b) und Gewindeeinschnitt 3 (mit erstem Gewindeeinschnitt 3a und zweitem Gewindeeinschnitt 3b) sind in der Figur 5 gekennzeichnet. Die Gewindeerhebung 4 bildet zusammen mit dem Gewindeeinschnitt 3 den Gewindegang.

Erfindungsgemäß ist die Klemmschraube 1 einschraubseitig mit einem Gewindeanschnitt 7 versehen. Dessen umfängliche Mantelfläche 23 ist in den Figuren 5, 7 und 8 durch eine Kreuzschraffierung kenntlich gemacht und parallel zur Mittellängsachse 6 der Klemmschraube 1 ausgebildet. Der Gewindeanschnitt 7 weist einen Übergangsabschnitt 24 sowie einen Teilbereich 25 mit konstantem radialem Abstand von der Mittellängsachse 6 auf. Der radiale Abstand der Mantelfläche 23 von der Mittellängsachse 6 entspricht auslaufseitig des Übergangsabschnitts 24, also beim Übergang 26 vom Übergangsabschnitt 24 zur nicht angeschnittenen Gewindeerhebung 4, dem Gewindeaußenradius. Der radiale Abstand der Mantelfläche 23 von der Mittellängsachse 6 einlaufseitig des Übergangsabschnitts 24, also am Übergang 27 vom Übergangsabschnitt 24 zum Teilbereich 25 mit konstantem Radius, ist kleiner oder gleich dem Gewindekernradius.

In Figur 6 sind der Übergangsabschnitt 24 und der Teilbereich 25 mit konstantem Radius gesondert kenntlich gemacht. Bei der gezeigten Ausführungsform erstreckt sich der Übergangsbereich 24 über einen Winkelabschnitt α von ca. 90°. Der Teilbereich 25 mit konstantem Radius erstreckt sich über einen Winkelabschnitt β von ca. 270°. In Fig. 6 ist ebenfalls die Änderung des Abstandes der Mantelfläche in radialer Richtung R von der Mittellängsachse 6 einlaufseitig gezeigt. Dies resultiert in unterschiedlichen Abständen von zwei sich gegenüber der Mittellängsachse 6 diametral gegenüberliegenden Punkten auf der radialen Außenfläche (siehe auch Fig. 5) der Klemmschraube 1 im Bereich der Stirnseite 22. Diese Abstände werden im Folgenden Durchmesser genannt. Ein in Fig. 6 gesehen horizontaler Durchmesser der Mantelfläche 23 an der Stirnseite 22 der Klemmschraube 1, also einem Abstand zwischen einem Punkt auf der Mantelfläche 23 im Übergangsbereich 24 und einem Punkt auf der Mantelfläche 23 in dem Teilbereich mit konstantem Radius 25, ist größer als ein in Fig. 6 gesehen vertikaler Durchmesser der Mantelfläche 23, also einem Abstand zwischen zwei Punkten auf der Mantelfläche 23 in dem Teilbereich mit konstantem Radius 25, an der Stirnseite 22 der Klemmschraube 1. Die Klemmschraube 1 kann dabei in einer ersten Stellung bzw. Einsetzstellung, bei der der kleinere Durchmesser (in Fig. 6 gesehen der vertikale Durchmesser) zwischen den zwei Flanken einer verformten Tulpe 14 (siehe Fig. 4) liegt, eingesetzt werden. Mit anderen Worten liegen an den Flanken der Tulpe 14 die Mantelflächen 23 des Teilbereichs mit konstantem Radius 25 an. Anschließend wird die Klemmschraube 1 um 90 Grad in eine zweite Stellung bzw. Spreizstellung eingedreht. Hierbei wird der Übergangsbereich 24 mit dem größer werdenden radialen Abstand der Mantelfläche 23 zwischen die beiden Flanken der Tulpe 14 eingedreht und die Flanken der Tulpe 14 gespreizt. Der Übergangsbereich 24 bzw. die radiale Außenfläche der ersten Gewindeerhebung 4a liegen an der Flanke der Tulpe 14 an.

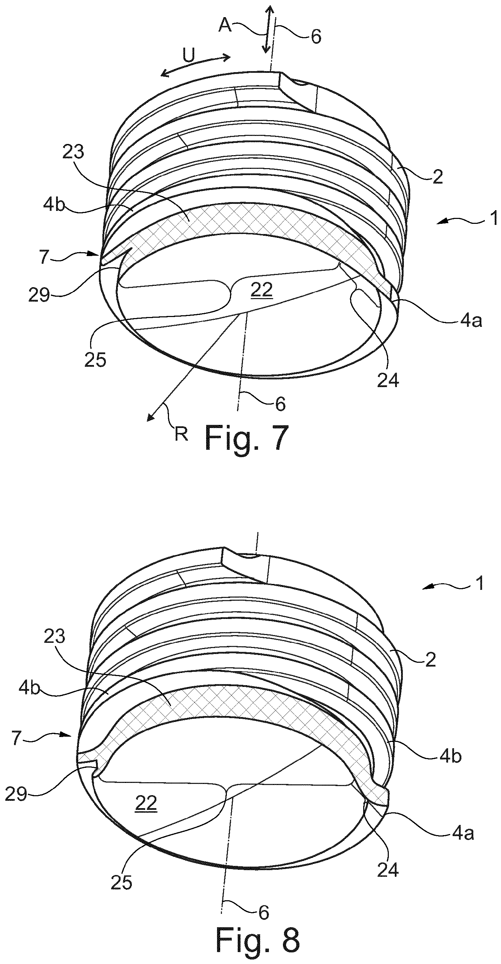

Fig. 7 zeigt eine Variante des Gewindeanschnitts 7 nach der Erfindung. Auch bei dieser ist die erste Gewindeerhebung 4a in radialer Richtung R angeschnitten, wobei der Übergangsabschnitt 24 zum nicht angeschnittenen Teil der ersten Gewindeerhebung 4a hin nahezu linear oder harmonisch auslaufend ausgebildet ist. Anders als bei der Ausführungsform der Figuren 5 und 6 folgt der Gewindeanschnitt 7 aber nicht der Steigung des Klemmschraubengewindes 2, sondern ist quer zur Mittellängsachse 6 ausgerichtet. Aus diesem Grund ist die zweite Gewindeerhebung 4b nicht nur in radialer Richtung R, sondern auch in axialer Richtung A angeschnitten. Es wird so ein zweiter Übergangsabschnitt 29 gebildet, der auf der dem Übergangsabschnitt 24 gegenüberliegenden Seite des Gewindeanschnitts 7 liegt und dem Übergangsabschnitt 24 gleicht. Die Ausführungsform der Figur 8 ist ähnlich, nur dass hier der Übergangsabschnitt 24 und der Übergangsabschnitt 29 bogenförmig ausgebildet sind. Bei den Ausführungsformen der Figuren 7 und 8 ist die beim Ansetzen und Einschrauben der Klemmschraube 1 besonders kritische erste Gewindeerhebung 4a in axiale Richtung nicht geschwächt und robust. Zwar ist die zweite Gewindeerhebung 4b in axialer Richtung A angeschnitten, aber diese ist in dieser Hinsicht weniger kritisch und die gezeigten Varianten bieten fertigungstechnische Vorteile bei der Ausbildung des Gewindeanschnitts 7.

Die Figuren 5, 7 und 8 zeigen jeweils deutlich, dass nach der Erfindung die Gewindeerhebung 4 durch den Gewindeanschnitt 7 in axialer Richtung A nicht verjüngt und damit geschwächt wird. Vielmehr ist die erste Gewindeerhebung 4a in radialer Richtung R angeschnitten, so dass sie durch den Gewindeanschnitt 7 in radiale Richtung R zur Mittellängsachse 6 der Klemmschraube 1 hin abgeflacht ist. Man erkennt, dass der Gewindeanschnitt 7 lediglich die erste Gewindeerhebung 4a in radialer Richtung R anschneidet, wobei hingegen die zweite Gewindeerhebung 4b nicht angeschnitten ist. Keine der Gewindeerhebungen 4a und 4b ist in axialer Richtung A angeschnitten. Umgekehrt betrachtet beginnt der Gewindeanschnitt 7 am Übergangsabschnitt 25 mit großer Höhe in axialer Richtung A und ist dann auslaufend ausgebildet und wird zur Stirnseite 22 hin immer dünner. Man kann also sagen, dass der Gewindeanschnitt der Steigung des Klemmschraubengewindes 2 folgt oder mit gleicher Steigung ausgebildet ist. Die kleinste Erstreckung 28 des Gewindeanschnitts 7 in axialer Richtung A ist in Figur 5 gekennzeichnet und beträgt nicht mehr als 0,5 mm.

Durch den Gewindeanschnitt 7 nach der Erfindung wird zum einen erreicht, dass sich die Verriegelungsschraube 1 auch dann einschrauben lässt, wenn der Tulpenkopf 14 durch von außen wirkende Kräfte elastisch nach innen verformt ist. Zum anderen kann so der Gewindeanschnitt 7 breit ausgebildet sein, so dass das Risiko einer Beschädigung des Anschnittes 7 und insbesondere der ersten Gewindeerhebung 4a beim Einschrauben reduziert ist.

### Bezugszeichenliste

- 1: Klemmschraube
- 2: Klemmschraubengewinde
- 3: Gewindeeinschnitt
- 3a: erster Gewindeeinschnitt
- 3b: zweiter Gewindeeinschnitt
- 4: Gewindeerhebung
- 4a: erste Gewindeerhebung
- 4b: zweite Gewindeerhebung
- 5: Klemmschraubengrundkörper
- 6: Mittellängsachse
- 7: Gewindeanschnitt
- 8: Absatz
- 9: Schulter
- 10: Gewindeflanke
- 11: Gewindeflanke
- 12: Spitze
- 13: Abschrägung
- 14: Tulpe, Aufnahmehülse
- 15: Pedikelschraube
- 16: Außengewinde, Knochengewinde
- 17: Werkzeugeingriff
- 18: Tulpengewinde
- 19: Loch
- 20: Hülsenwandabschnitt
- 21: Hülsenwandabschnitt
- 22: Stirnseite
- 23: Mantelfläche
- 24: Übergangsabschnitt
- 25: Teilbereich mit konstantem Radius
- 26: Übergang von 4 nach 24
- 27: Übergang von 24 nach 25
- 28: kleinste Erstreckung
- 29: zweiter Übergangsabschnitt
- A: Axialrichtung
- R: Radialrichtung
- U: umfängliche Richtung
- α: Winkelabschnitt
- β: Winkelabschnitt

## Patentansprüche

1. Klemmschraube (1) für ein Knochenschraubensystem, welches eine Knochenschraube (15) mit einer Tulpe (14), die eine Aufnahme für einen Längsträger zur chirurgischen Verbindung benachbarter Knochenschrauben (15) ausbildet, und mit einem Tulpengewinde (18) versehen ist, aufweist, wobei
die Klemmschraube ein Klemmschraubengewinde (2) zur klemmenden Verriegelung des Längsträgers in der Aufnahme aufweist, wobei
das Klemmschraubengewinde (2) einlaufseitig einen radialen Gewindeanschnitt aufweist, an dem das Klemmschraubengewinde (2) in radialer Richtung reduziert oder zurückgenommen ist, wobei der Gewindeanschnitt (7) eine Mantelfläche (23) aufweist, und
der Gewindeanschnitt (7) einen Übergangsabschnitt (24) aufweist, in dem die Mantelfläche (23) einen variierenden radialen Abstand in radialer Richtung (R) von der Längsachse (6) der Klemmschraube (1) aufweist,
**dadurch gekennzeichnet, dass** der radiale Abstand der Mantelfläche (23) von der Längsachse (6) auslaufseitig des Übergangsabschnitts (24) dem Gewindeaußenradius entspricht und der radiale Abstand der Mantelfläche (23) von der Längsachse (6) einlaufseitig des Übergangsabschnitts (24) kleiner als der Gewindekernradius ist.

2. Klemmschraube (1) nach Anspruch 1, **dadurch gekennzeichnet, dass** sich der radiale Abstand der Mantelfläche (23) des Übergangsabschnitts (24) von der Längsachse (6) im umfänglichen Verlauf kontinuierlich und/oder konstant ändert, insbesondere linear oder bogenförmig oder kreisbogenförmig ausgebildet ist.

3. Klemmschraube (1) nach einem der Ansprüche 1 oder 2, **dadurch gekennzeichnet, dass** sich der Übergangsabschnitt (24) in Umfangsrichtung über einen Winkelabschnitt α zwischen ca. 20° und ca. 180°, vorzugsweise zwischen ca. 40° und ca. 135°, bevorzugter zwischen ca. 60° und ca. 90° erstreckt.

4. Klemmschraube (1) nach einem der vorstehenden Ansprüche, **dadurch gekennzeichnet, dass** der Gewindeanschnitt (7) in einem Teilbereich (25) teilzylinderförmig mit konstantem radialen Abstand von der Längsachse (6) ausgebildet ist.

5. Klemmschraube (1) nach Anspruch 4, **dadurch gekennzeichnet, dass** der radiale Abstand der Mantelfläche (23) in dem Teilbereich (25) mit konstantem radialen Abstand kleiner oder gleich dem Gewindekernradius ist.

6. Klemmschraube (1) nach Anspruch 4 oder 5, **dadurch gekennzeichnet, dass** sich der Teilbereich (25) mit konstantem radialen Abstand in Umfangsrichtung über einen Winkelbereich β von mindestens 90°, vorzugsweise von 90° bis 180° erstreckt.

7. Klemmschraube (1) nach einem der vorstehenden Ansprüche, **dadurch gekennzeichnet, dass** der Gewindeanschnitt (7) dort endet, wo die Gewindespitze des Klemmschraubengewindes (2) einlaufseitig ihre normale Breite annimmt.

8. Klemmschraube (1) nach einem der vorstehenden Ansprüche, **dadurch gekennzeichnet, dass** der Gewindeanschnitt (7) der Steigung des Klemmschraubengewindes (2) folgend ausgebildet ist.

9. Knochenschraubensystem, mit:
einer Knochenschraube (15) mit einer Tulpe (14), die eine Aufnahme für einen Längsträger zur chirurgischen Verbindung benachbarter Knochenschrauben (15) ausbildet und mit einem Tulpengewinde (18) versehen ist, und
einer Klemmschraube (1) nach einem der vorstehenden Ansprüche zum Einschrauben in das Tulpengewinde (18) und zur klemmenden Verriegelung des Längsträgers in der Aufnahme.

## Claims

1. A clamping screw (1) for a bone screw system which includes a bone screw (15) comprising a tulip (14) which forms a seat for a longitudinal support for surgical connection of adjacent bone screws (15) and is provided with a tulip thread (18), wherein
the clamping screw comprises a clamping screw thread (2) for locking the longitudinal support by clamping in the seat, wherein
the clamping screw thread (2) on the run-in side includes a radial thread cut (7) at which the clamping screw thread (2) is reduced or withdrawn in the radial direction, wherein the thread cut (7) has a circumferential surface (23) and
the thread cut (7) has a transition section (24) in which the circumferential surface (23) has a varying radial distance in the radial direction (R) from the longitudinal axis (6) of the clamping screw (1),
**characterized in that** the radial distance of the circumferential surface (23) from the longitudinal axis (6) on the run-out side of the transition section (24) corresponds to the outer thread radius and the radial distance of the circumferential surface (23) from the longitudinal axis (6) on the run-in side of the transition section (24) is smaller than the thread core radius.

2. The clamping screw (1) according to claim 1, **characterized in that** the radial distance of the circumferential surface (23) of the transition section (24) from the longitudinal axis (6) in the circumferential extension varies continuously and/or constantly, especially is configured to be linear or curved or circular arc-shaped.

3. The clamping screw (1) according to any one of the claims 1 or 2, **characterized in that** the transition section (24) in the circumferential direction extends over an angular portion α between approx. 20° and approx. 180°, preferably between approx. 40° and approx. 135°, more preferred between approx. 60° and approx. 90°.

4. The clamping screw (1) according to any one of the preceding claims, **characterized in that** the thread cut (7) is configured in a subarea (25) in pitch cylinder shape having a constant radial distance from the longitudinal axis (6).

5. The clamping screw (1) according to claim 4, **characterized in that** the radial distance of the circumferential surface (23) in the subarea (25) having the constant radial distance is smaller than or equal to the thread core radius.

6. The clamping screw (1) according to claim 4 or 5, **characterized in that** the subarea (25) having a constant radial distance extends in the peripheral direction over an angular area β of at least 90°, preferably from 90° to 180°.

7. The clamping screw (1) according to one of the preceding claims, **characterized in that** the thread cut (7) ends where the thread crest of the clamping screw thread (2) on the run-in side adopts its normal width.

8. The clamping screw (1) according to one of the preceding claims, **characterized in that** the thread cut (7) is configured to follow the elevation of the clamping screw thread (2).

9. A bone screw system comprising:
a bone screw (15) having a tulip (14) which forms a seat for a longitudinal support for surgical connection of adjacent bone screws (15) and is provided with a tulip thread (18), and
a clamping screw (1) according to any one of the preceding claims for screwing into the tulip thread (18) and for locking the longitudinal support by clamping within the seat.

## Revendications

1. Vis de serrage (1) destinée à un système de vis d'ostéosynthèse, lequel présente une vis d'ostéosynthèse (15) munie d'une tulipe (14), qui forme un logement pour un support longitudinal destiné à l'assemblage chirurgical de vis d'ostéosynthèse (15) voisines et qui est dotée d'un filet de tulipe (18),
la vis de serrage présentant un filet de vis de serrage (2) pour réaliser le verrouillage par serrage du support longitudinal dans le logement,
le filet de vis de serrage (2) présentant en amont une partie filetée radiale, sur laquelle est diminué ou retiré le filet de vis de serrage (2) dans le sens radial, la partie filetée (7) présentant une surface latérale (23), et
la partie filetée (7) présentant une section de transition (24), dans laquelle la surface latérale (23) présente une distance radiale variable dans le sens radial (R) par rapport à l'axe longitudinal (6) de la vis de serrage (1),
**caractérisée en ce que** la distance radiale de la surface latérale (23) par rapport à l'axe longitudinal (6) en aval de la partie de transition (24) correspond au rayon externe de la partie filetée et la distance radiale de la surface latérale (23) par rapport à l'axe longitudinal (6) en amont de la section de transition (24) est inférieure au rayon central de la partie filetée.

2. Vis de serrage (1) selon la revendication 1, **caractérisée en ce que** la distance radiale de la surface latérale (23) de la section de transition (24) par rapport à l'axe longitudinal (6) varie continuellement et/ou constamment suivant un tracé circonférentiel, est en particulier de forme linéaire ou en forme d'arc ou en arc de cercle.

3. Vis de serrage (1) selon l'une quelconque des revendications 1 ou 2, **caractérisé en ce que** la section de transition (24) s'étend dans le sens circonférentiel sur une section angulaire α comprise entre environ 20° et environ 180°, de préférence entre environ 40° et environ 135 °, de manière davantage préférée entre environ 60° et environ 90°.

4. Vis de serrage (1) selon l'une quelconque des revendications précédentes, **caractérisée en ce que** la partie filetée (7) est formée dans une zone partielle (25) en forme de cylindre partiel à distance radiale constante par rapport à l'axe longitudinal (6).

5. Vis de serrage (1) selon la revendication 4, **caractérisée en ce que** la distance radiale de la surface latérale (23) dans la zone partielle (25) à distance radiale constante est inférieure ou égale au rayon central de la partie filetée.

6. Vis de serrage (1) selon la revendication 4 ou 5, **caractérisée en ce que** la zone partielle (25) s'étend à distance radiale constante dans le sens circonférentiel sur une plage angulaire β d'au moins 90°, de préférence de 90° à 180°.

7. Vis de serrage (1) selon l'une quelconque des revendications précédentes, **caractérisée en ce que** la partie filetée (7) se termine où la pointe filetée du filet de vis de serrage (2) prend en amont sa largeur normale.

8. Vis de serrage (1) selon l'une quelconque des revendications précédentes, **caractérisée en ce que** la partie filetée (7) est formée en suivant l'inclinaison du filet de vis de serrage (2).

9. Système de vis d'ostéosynthèse, comprenant :
une vis d'ostéosynthèse (15) munie d'une tulipe (14), qui forme un logement pour un support longitudinal destiné à l'assemblage chirurgical de vis d'ostéosynthèse (15) voisines et qui est dotée d'un filet de tulipe (18), et
une vis de serrage (1) selon l'une quelconque des revendications précédentes destinée à être vissée dans un filet de tulipe (18) et pour réaliser le verrouillage par serrage du support longitudinal dans le logement.
